# EUROPÄISCHE PATENTSCHRIFT

(11) **EP 1 508 563 B1**
(45) Veröffentlichungstag und Bekanntmachung des Hinweises auf die Patenterteilung: **17.04.2013**
(21) Anmeldenummer: 04002754.2
(22) Anmeldetag: 07.02.2004
(51) Int. Cl.: C07C 201/08, C07C 201/16, C07C 205/06

(54) **Verfahren zur zweistufigen Herstellung von Dinitrotoluol**
Two step process for the production of Dinitrotoluene
Procédé pour la préparation de dinitrotoluene en deux étapes

(30) Priorität: 20.02.2003 DE 10307140
(43) Veröffentlichungstag der Anmeldung: 23.02.2005
(73) Patentinhaber: Bayer MaterialScience AG, 51368 Leverkusen (DE)
(72) Erfinder: Dieterich, Erwin, Dr., 50769 Köln (DE); Hielscher, Anke, 51373 Leverkusen (DE); Keggenhoff, Berthold, Dr., 47839 Krefeld (DE); Keller-Killewald, Manfred, 25541 Brunsbüttel (DE); Münnig, Jürgen, 41564 Kaarst (DE); Wastian, Dietmar, 41542 Dormagen (DE)

(56) Entgegenhaltungen:
- EP-A- 0 903 336
- DE-A- 4 309 140

## Beschreibung

Die Erfindung betrifft ein Verfahren zur Nitrierung von Toluol mit Nitriersäure zu Dinitrotoluol (DNT) in einem zweistufigen Prozess, wobei die erste Stufe adiabatisch und die zweite Stufe isotherm durchgeführt wird.

Dinitrotoluol (DNT) ist das Vorprodukt von Toluylendiisocyanat, das als Rohstoff für die Herstellung von Polyurethanen eingesetzt wird. Das technisch übliche Verfahren zur Herstellung von Dinitrotoluol ist die isotherme zweistufige Umsetzung (EP 0 903 336) von Toluol mit, Nitriersäure, einem Gemisch aus Salpeter- und Schwefelsäure Ullmanns Encyklopädie der technischen Chemie, 4. Auflage, Band 17, Seite 392, Verlag Chemie, Weinheim 1979. Hierbei wird zunächst ein Isomeren-Gemisch von Mononitrotoluol (MNT) hergestellt, welches in einem zweiten separaten Verfahrensschritt zu Dinitrotoluolen umgesetzt wird. Das Verfahren hat allerdings den Nachteil, dass die in beiden Stufen anfallende Säurephase (im wesentlichen Schwefelsäure) energieaufwendig vom aufgenommenen Wasser befreit werden muss.

Es ist ebenfalls bekannt, Toluol einstufig auf adiabatischem Weg zu Dinitrotoluol umzusetzen (EP-A-597 361). Hierbei wird Toluol mit mindestens 2 Äquivalenten einer Nitriersäure bestimmter Zusammensetzung auf adiabatischem Wege umgesetzt, wobei eine Endtemperatur von über 120°C erreicht wird. Nach Trennung der Phasen bei dieser Temperatur wird die Säurephase einer Aufkonzentrierung (Flashverdampfung im Vakuum) zugeführt, wobei der Wärmeinhalt der Säure zur Aufkonzentrierung genutzt wird. Die aufkonzentrierte Säure wird mit Salpetersäure aufgestockt und in den Prozess zurückgeführt.

Bei diesem Verfahren ergibt sich jedoch die Schwierigkeit, dass bei der Flashverdampfung zusammen mit dem abdestillierenden Wasser ein gewisser, in der Säure gelöster Anteil an DNT mit in die Gasphase übergeht, welcher dann bei der Kondensation der Brüden unter den Kondensationsbedingungen des Wassers fest wird (Festpunkt des Isomerengemisches von 52 - 58°C) und den Wärmetauscher belegt. Außerdem werden bei der Prozessführung Temperaturen erreicht, bei denen das DNT in Gegenwart von Nebenprodukten nicht dauerhaft stabil ist. Zur Gewährleistung der Prozesssicherheit darf die zulässige Verweilzeit stark DNT-haltiger Stoffströme bei hohen Temperaturen nicht überschritten werden. Das erfordert einen erheblichen Aufwand für Sicherheitseinrichtungen.

Für das Problem der Störung der Kondensation durch in den Brüden enthaltenes DNT sind verschiedene Lösungen vorgeschlagen worden, wie wechselseitig betriebene, getaktete Wärmetauscher, Misch- oder Einspritzkondensatoren (R.A. Vauck, H. A. Müller, Grundoperationen chemischer Verfahrenstechnik, 5.Auflage, VEB Leipzig 1962, S. 447). Ferner beschreibt die EP-A-0696569 ein Verfahren zur adiabatischen Herstellung von DNT, wobei gezielt mindestens 5 % MNT im Reaktionsprodukt der adiabaten Nitrierung verbleiben, um die Belegung der Wärmetauscher zu verhindern.

Alle diese Verfahren sind entweder technisch oder energetisch aufwendig oder sie erlauben nicht, DNT mit einem sehr geringen Anteil an MNT zu erzeugen. Außerdem bleibt bei der adiabatischen Reaktionsführung das Problem der hohen Temperaturbelastung von DNT oder stark DNT-haltigen Medien.

Aufgabe der vorliegenden Erfindung war daher, ein technisch einfaches Verfahren zur Herstellung von Dinitrotoluol durch Nitrierung von Toluol zur Verfügung zu stellen, bei dem man zumindest einen Teil der anfallenden Reaktionswärme zur Aufkonzentrierung der Absäure nutzen kann und gleichzeitig DNT und DNT-haltige Medien keinen sicherheitstechnisch bedenklichen Temperaturen aussetzt.

Die Erfindung betrifft ein Verfahren zur Herstellung von Dinitrotoluol durch zweistufige Nitrierung von Toluol, bei dem man
a) in einer ersten Stufe Toluol mit Nitriersäure adiabatisch umsetzt, wobei das Toluol zu mindestens 90 %, bevorzugt zu mindestens 98 %, abreagiert und wobei maximal 70 %, bevorzugt bis zu 50 % des eingesetzten Toluols zu Dinitrotoluol reagieren, anschließend die Mononitrotoluol enthaltende organische Phase und die wässrige, Schwefelsäure enthaltende Säurephase trennt, die wässrige, Schwefelsäure enthaltende Säurephase durch Entspannungsverdampfung aufkonzentriert und die dabei erhaltene aufkonzentrierte Schwefelsäure in die Reaktion der ersten Stufe und / oder die Reaktion der zweiten Stufe und / oder die Aufkonzentrierung der zweiten Stufe zurückführt, und
b) in einer zweiten Stufe die Mononitrotoluol enthaltende organische Phase aus der ersten Stufe mit Nitriersäure isotherm vollständig umsetzt, anschließend die organische Phase und die wässrige, Schwefelsäure enthaltende Säurephase trennt und die wässrige, Schwefelsäure enthaltende Säurephase durch Vakuumverdampfung aufkonzentriert und die dabei erhaltene aufkonzentrierte Schwefelsäure in die Reaktion der ersten Stufe und / oder der zweiten Stufe zurückführt.

Bei dem erfindungsgemäßen Verfahren lässt man in der ersten Stufe bevorzugt 98 % des Toluols abreagieren wobei sich bevorzugt bis zu 50 %, besonders bevorzugt bis zu 30 % des eingesetzten Toluols zu Dinitrotoluol umsetzen.

Die erste, adiabatische Stufe kann in jedem geeigneten Reaktor ausgeführt werden. Bevorzugt werden jedoch Rohrreaktoren eingesetzt, deren Länge von 2 bis 20 m und deren Durchmesser von 25 bis 2000 mm betragen können. Die Länge und der Durchmesser werden dabei vorzugsweise so gewählt, dass die mittlere Verweilzeit 10 bis 300 Sekunden beträgt. Es können Rohre ohne, oder bevorzugt Rohre mit geeigneten Einbauten zur Dispergierung des zweiphasigen Gemischs eingesetzt werden. Vorteilhaft verwendbare Einbauten sind z.B. Siebböden oder statische Mischelemente, wie sie z.B. in EP-A-0708076 oder EP-A-0489211 beschrieben sind.

Die eingesetzte Nitriersäure enthält bevorzugt 80 bis 100 Gewichtsprozent (Gew.-%) anorganische Bestandteile, die sich im Wesentlichen aus 50 bis 90 Gew.-% Schwefelsäure, 1 bis 20 Gew.-% Salpetersäure und mindestens 5 Gew.-% Wasser zusammensetzen. Das Molverhältnis von Salpetersäure:Toluol beträgt bevorzugt mindestens 0,7:1 und höchstens 1,8:1, besonders bevorzugt höchstens 1,5:1. Die eingesetzte Salpetersäure kann eine Konzentration von 50 - 100 Gew.-% haben. Bevorzugt wird 62 - 70 gew.-%ige Salpetersäure eingesetzt.

Die Nitriersäure wird am Reaktoreingang mit Toluol vermischt, was über eine einfache Leitungszuführung oder spezielle Mischorgane, wie Düsen oder spezielle statische Mischer, erfolgen kann. Bevorzugt wird das Toluol über ein Dispergierorgan, beispielsweise ein Strahldispergator, in der Nitriersäure dispergiert.

Die Mischung hat bei Reaktoreintritt bevorzugt eine Temperatur von 80 bis 120°C, die zum Reaktoraustritt durch die Reaktionswärme auf 120 bis 170°C steigt. Der Temperaturanstieg beträgt dabei vorzugsweise 20 bis 80°C.

Nach Austritt aus dem Reaktor wird die zweiphasige Mischung in die wässrige, Schwefelsäure enthaltende Säurephase und die organische, Mononitrotoluol enthaltende Phase getrennt, was bevorzugt in statischen Phasenscheidern geschieht.

Die organische Phase kann dann, ganz oder anteilig, zum Extrahieren der Absäure der zweiten Stufe eingesetzt und dann der zweiten Nitrierstufe zugeführt werden. Sie enthält bevorzugt maximal 10 Gew.-%, besonders bevorzugt weniger als 2 Gew.-% Toluol, und bevorzugt maximal 70 Gew.-%, besonders bevorzugt bis zu 50 Gew.-% und ganz besonders bevorzugt bis zu 30 Gew.-% DNT.

Die wässrige Säurephase (Absäure) wird durch Entspannungsverdampfung bevorzugt bei einem Druck von 10 bis 400 mbar, gegebenenfalls auch unter weiterer Wärmezufuhr über geeignete Wärmetauscher von bevorzugt 60 - 110 % des bei der Reaktion entstandenen und mit der Salpetersäure zugeführten Wassers befreit und anschließend zum Reaktoreintritt zurückgeführt. Bevorzugt wird genau die bei der Reaktion entstandene und mit der Salpetersäure zugeführte Menge an Wasser verdampft. Abweichungen von diesem bevorzugten Gleichgewichtszustand können aber durch Austausch von Schwefelsäuremengen aus dem Kreislauf der ersten Stufe mit Absäure aus dem Kreislauf der zweiten Stufe und / oder mit aufkonzentrierter Schwefelsäure der Säureaufkonzentrierung der zweiten Stufe ausgeglichen werden.

In einer weiteren Ausführungsform des Verfahrens werden die Stufen der Entspannungsverdampfung und Phasentrennung in umgekehrter Reihenfolge ausgeführt, d. h. das zweiphasige Gemisch wird der Entspannungsverdampfung unter den o.g. Bedingungen unterworfen, wonach das abgekühlte Gemisch in die Säure- und organische Phase getrennt werden.

Die aufgrund der Wasserdampfflüchtigkeit mit in die Brüden überführten organischen Bestandteile werden zusammen mit dem Wasser kondensiert. Aufgrund des hohen MNT-Anteils besteht keine Gefahr dass sich im Brüdenkondensator feste DNT- oder DNT/MNT-Beläge bilden. Das zweiphasige Brüdenkondensat wird in die organische und wässrige Phase getrennt, was bevorzugt in statischen Abscheidern erfolgen kann. Die organische Phase kann, ganz oder anteilig, zum Extrahieren' der Absäure der zweiten Stufe eingesetzt und dann der zweiten Nitrierstufe zugeführt werden.

Die zweite, isotherme Stufe wird in einem geeigneten Reaktor oder einer Serie von Reaktoren mit Kühlvorrichtung ausgeführt. Bevorzugt werden Rührkesselreaktoren oder Schleifenreaktoren mit Wärmetauschern, wie in Ullmanns Encyklopädie der technischen Chemie, 4. Auflage, Band 17, Seite 392, Verlag Chemie, Weinheim 1979 beschrieben verwendet. Die Reaktionstemperatur wird dabei üblicherweise durch Wärmeabfuhr mit Kühlwasser eingestellt und liegt vorzugsweise bei 60 bis 95°C, besonders bevorzugt bei 60 bis 80°C. Die Verweilzeit beträgt vorzugsweise 1 bis 10 min.

In den Reaktor bzw. die Reaktorkaskade wird die organische Phase aus der ersten Stufe, die Mononitrotoluol enthält und zuvor ganz oder teilweise zur Extraktion der wässrigen Säurephase aus der zweiten Stufe eingesetzt worden sein kann, gefördert und mit Nitriersäure vermischt. Die Nitriersäure wird vorzugsweise durch Vermischung von aufkonzentrierter Schwefelsäure mit einer Konzentration von 83 bis 98 Gew.-%, besonders bevorzugt 85 bis 98 Gew.-% und Salpetersäure hergestellt. Die Menge an Nitriersäure in der zweiten Stufe wird dabei so gewählt, dass die Summe der in der ersten und der zweiten Stufe eingesetzten Salpetersäure 1,9 = 2,2 mol, bevorzugt 2,0 - 2,05 mol pro mol eingesetztem Toluol ergibt. Die Konzentration der eingesetzten Salpetersäure ist bevorzugt gleich der in der ersten Stufe verwendeten.

Die aus der Reaktion der zweiten Stufe austretende, zweiphasige Mischung wird in die organische und die wässrige, Schwefelsäure enthaltende Säurephase getrennt, was mittels Zentrifugen, oder, bevorzugt, in statischen Abscheidern erfolgen kann. Die organische Phase wird in geeigneter Weise, z.B. durch Extraktion mit Wasser und / oder Sodalösung, von Säurespuren befreit und so das Endprodukt Dinitrotoluol gewonnen.

Die erhaltene Säurephase (Absäure) wird bevorzugt zunächst mit MNT-haltiger organischer Phase aus der ersten Stufe extrahiert, um den Gehalt an gelöstem DNT zu reduzieren. Dazu kann das gesamte oder ein Teil des organischen Brüdenkondensats aus der ersten Stufe und / oder die gesamte oder ein Teil der organischen Phase aus der ersten Stufe genutzt werden. Dies kann einstufig oder mehrstufig erfolgen, wobei Mischer-Scheider-Einheiten oder auch Rühr-Extraktionskolonnen verwendet werden können. Vor oder nach der Extraktion kann ein Teil der Absäure abgezweigt und der Schwefelsäure der ersten Nitrierstufe vor oder nach der Aufkonzentrierung hinzugefügt werden.

Nach der Extraktion wird die Absäure der Dihitrierung in einer geeigneten Destillationsanlage von dem bei der Reaktion entstandenen und mit der Salpetersäure zugeführten Wasser befreit. Ein geeignetes Destillierverfahren ist z.B. in DE-A-19636191 beschrieben. Die in die Destillationsanlage einlaufende Absäure hat vorzugsweise eine Konzentration von 75 bis 90 Gew.-% Schwefelsäure und wird vorzugsweise auf eine Konzentration von 83 bis 98 Gew.-%, besonders bevorzugt 85 bis 98 Gew.-% Schwefelsäure aufkonzentriert.

Dieser Säureaufkonzentrierung können zusätzlich, um den Schwefelsäurehaushalt in der ersten Stufe auszugleichen, bis zu 30 % der im adiabatisch betriebenen Teil (der ersten Stufe) anfallenden Absäure, bevorzugt Schwefelsäure nach der Flashverdampfung, zugeführt werden. Zum Ausgleich der Schwefelsäurebilanz in der isotherm betriebenen Stufe, muss dann eine entsprechende Menge der die Säureaufkonzentrierung verlassenden Schwefelsäure dem Säurekreislauf der ersten Stufe zugegeben werden.

Schwefelsäureverluste der beiden Reaktionsstufen können mit kommerziell erhältlicher 80 bis 100 Gew.-%, bevorzugt 90 bis 100 Gew.-% frischer Schwefelsäure ersetzt werden.

Das erfindungsgemäße Verfahren wird nachfolgend anhand der Figuren näher erläutert.

Es zeigen
- Figur 1: eine schematische Darstellung des Verfahrens, bei dem zuerst die organische Phase und die wässrige Säurephase aus der ersten Stufe getrennt werden und danach die wässrige Säurephase durch Entspannungsverdampfung aufkonzentriert wird.
- Figur 2: eine schematische Darstellung des Verfahrens, bei dem zuerst die organische Phase und die wässrige Säurephase gemeinsam die Entspannungsverdampfung durchlaufen und dann die organische Phase und die aufkonzentrierte wässrige Säurephase getrennt werden.
- Figur 3: eine schematische Darstellung des Verfahrens, bei dem zuerst die organische Phase und die wässrige Säurephase gemeinsam die Entspannungsverdampfung durchlaufen und dann die organische Phase und die aufkonzentrierte wässrige Säurephase getrennt werden und bei dem die wässrige Säurephase aus der zweiten Stufe mit dem organischen Brüdenkondensat aus der ersten Stufe extrahiert wird.

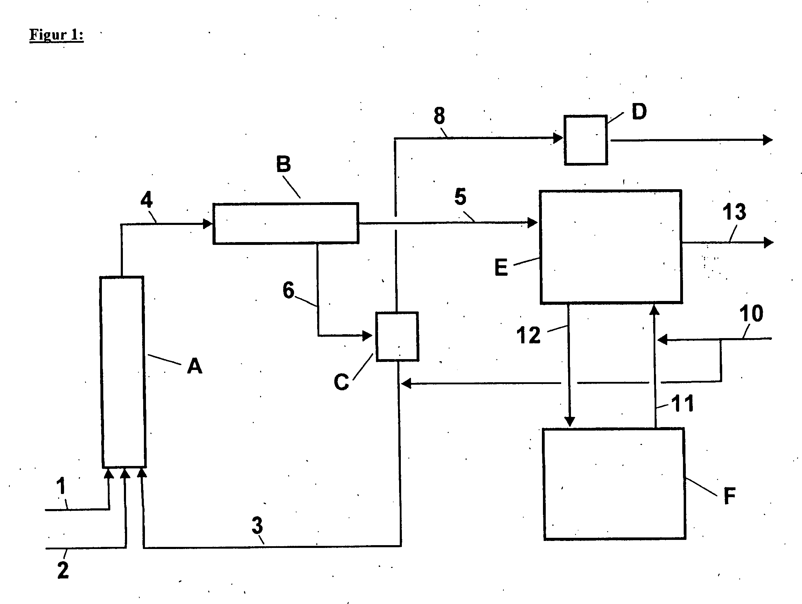

Eine Ausführungsform des erfindungsgemäßen Verfahrens ist in Figur 1 schematisch dargestellt. Dabei bedeutet A den Reaktor der ersten Stufe, B den Phasenabscheider des Reaktionsgemisches, C den Entspannungsverdampfer (Flashverdampfer mit Wärmetauscher im Sumpf) der wässrigen Säurephase der ersten Stufe, D den Kondensator des Entspannungsverdampfers, E den Reaktor und den Phasenabscheider der zweiten Stufe und F den Vakuumverdampfer für die Aufkonzentrierung der wässrigen Säurephase aus der zweiten Stufe. Toluol (Strom 1), Salpetersäure (Strom 2) und zurückgeführte, aufkonzentrierte Schwefelsäure (Strom 3) werden dem Reaktor A zugeführt und vermischt. Das erhaltene zweiphasige Reaktionsgemisch (Strom 4) wird anschließend in dem Phasenabscheider B in eine organische Phase (Strom 5) und eine wässrige Säurephase (Strom 6) getrennt. Die wässrige Säurephase (Strom 6) wird in dem Entspannungsverdampfer C aufkonzentriert. Die im Entspannungsverdampfer C entstehenden Brüden (Strom 8) werden im Kondensator D kondensiert und können aus dem Prozess ausgeschleust werden. Die aufkonzentrierte wässrige Säurephase (im Wesentlichen Schwefelsäure) (Strom 3) wird dem Reaktor A der ersten Stufe wieder zugeführt. Die organische Phase. (Strom 5) wird in den Reaktor E der zweiten Stufe eingeleitet und reagiert dort mit Nitriersäure zu Dinitrotoluol. Die Trennung der organischen Phase, die Dinitrotoluol enthält, und der wässrigen Säurephase ist nicht als eigener Schritt in Figur 1 dargestellt. Die organische Phase wird als Strom 13 ausgeschleust und weiter aufgearbeitet. Die wässrige Säurephase 12 (Absäure) wird in den Vakuumverdampfer F der zweiten Stufe gefördert, wo die Aufkonzentrierung der Absäüre stattfindet. Die aufkonzentrierte, wässrige Säurephase wird dann als Strom 11 wieder in den Eintritt in den Reaktor E zurückgerührt. Um Verluste an Schwefelsäure auszugleichen, wird frische Schwefelsäure (Strom 10) zugegeben. Die Zugabe erfolgt in die Ströme der aufkonzentrierten Schwefelsäure 3 und 11.

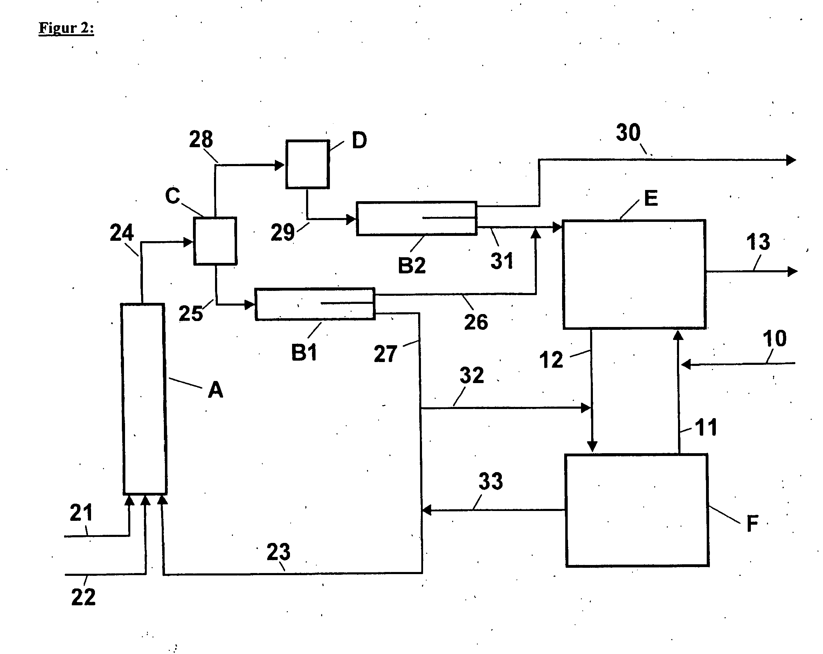

Figur 2 zeigt eine alternative Ausführungsform des erfindungsgemäßen Verfahrens. Toluol (Strom 21), Salpetersäure (Strom 22) und aufkonzentrierte Schwefelsäure (Strom 23) werden im Reaktor A zu einem Gemisch aus Mononitrotoluol und Dinitrotoluol umgesetzt. Das erhaltene zweiphasige Reaktionsgemisch (Strom 24) wird anschließend in den Entspannungsverdampfer C geleitet. Die dabei erhaltenen Brüden (Strom 28) werden im Kondensator D kondensiert und das Kondensat (Strom 29) dem Phasenabscheider B2 zugeleitet und dort in eine organische Phase (Strom 31) und eine wässrige Phase (Strom 30) aufgetrennt. Die wässrige Phase (Strom 30) wird aus dem Prozess ausgeschleust. Die in dem Entspannungsverdampfer C erhaltene flüssige Phase (aufkonzentrierte Schwefelsäure und organische Phase) (Strom 25) wird in dem Phasenabscheider B1 in eine organische Phase (Strom 26) und eine wässrige Säurephase (Strom 27) aufgetrennt. Ein Teil der aufkonzentrierten Schwefelsäure (Strom 32) wird zusammen mit Strom 12 in den Vakuumverdampfer F der zweiten Stufe gefördert. Die restliche Menge aus Strom 27 wird mit aufkonzentrierter Schwefelsäure (Strom 33) aus der zweiten Stufe zusammengeführt und als Strom 23 dem Reaktor A der ersten Stufe wieder zugeführt. Die organischen Phasen aus den Phasenabscheidem B1 (Strom 26) und B2 (Strom 31) werden vereinigt und dem Reaktor E der zweiten Stufe zugeführt. Die Mononitrotoluol enthaltenden organischen Phasen (Strom 26+31) werden dort mit Nitriersäure zu Dinitrotoluol umgesetzt. Die Trennung der organischen Phase, die Dinitrotoluol enthält, und der wässrigen Säurephase ist nicht als eigener Schritt in Figur 2 dargestellt. Die organische Phase wird als Strom 13 ausgeschleust und weiter aufgearbeitet. Die wässrige Säurephase 12 wird in den Vakuumverdampfer F der zweiten Stufe gefördert, wo die Aufkonzentrierung der Absäure stattfindet. Die aufkonzentrierte Schwefelsäure wird dann als Strom 11 wieder in den Eintritt von Reaktor E zurückgeführt. Um Verluste an Schwefelsäure auszugleichen, wird frische Schwefelsäure (Strom 10) zugegeben. Die Zugabe erfolgt dabei in den Säürestrom 11 hinein.

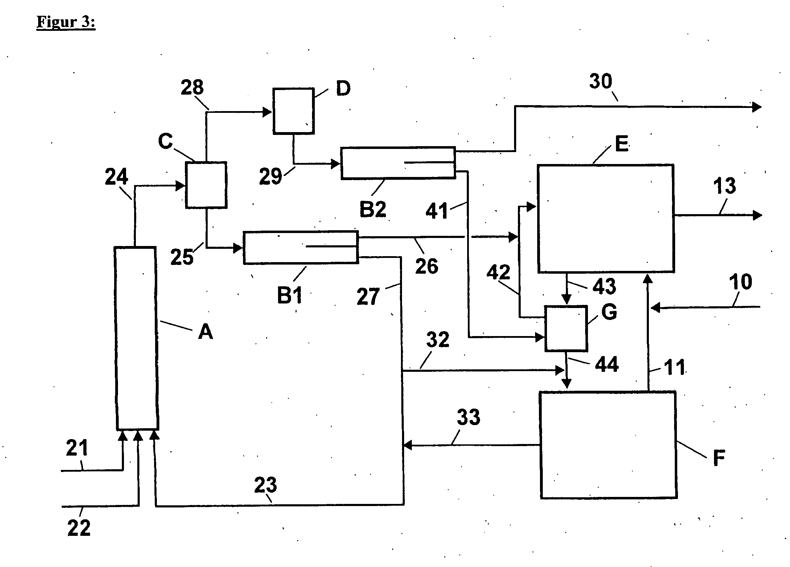

Figur 3 zeigt eine besondere Ausführung der in Figur 2 gezeigten Verfahrensvariante, die um eine Extraktionsstufe ergänzt ist. In dem Extraktionsapparat G wird die wässrige Säurephase (Strom 43) aus der zweiten Stufe mit dem organischen Brüdenkondensat (Strom 41) aus der ersten Stufe extrahiert. Der DNT-Gehalt der wässrige Säurephase (Strom 44) ist dadurch reduziert. Die organische Phase aus dem Phasenabscheider B 1 (Strom 26) und dem Extraktor G (Strom 42) werden vereinigt und dem Reaktor E der zweiten Stufe zugeführt. Im übrigen haben die Bezugszeichen in Figur 3 die gleiche Bedeutung wie in Figur 2.

### Beispiele

### Beispiel 1

Es wurde eine Apparatur gemäß Figur 1 verwendet. Die Temperaturen und Zusammensetzungen der Stoffströme sind in Tabelle 1 zusammengefasst. In der diabat betriebenen ersten Stufe wurden 50,6 kg/h Toluol (Strom 1), 63,0 kg/h 68 gewichtsprozentige (gew.-%ige) Salpetersäure (Strom 2) und 1066,6 kg/h 76,8 gew.-%ige aufkonzentrierte Absäure (Strom 3) am Eingang des Reaktors A (Abmessungen Länge (L) = 5 m, Durchmesser (D) = 25/80 mm) intensiv gemischt. In dem mit Lochscheiben zur Redispergierung ausgerüstete Rohrreaktor stieg die Temperatur auf 131 °C. Bei einer mittleren Verweilzeit von 15 min. wurde das Reaktionsgemisch (Strom 4) in dem Phasentrenner B in 78,1 kg/h einer organischen Phase (Strom 5) und 1102,1 kg/h einer wässrigen Säurephase (Strom 6) getrennt. Die 74,5 gew.-%ige Absäure wurde in dem bei 40 mbar betriebenen Flashverdampfer C auf einen Schwefelsäuregehalt von 76,8 Gew.-% aufkonzentriert. Dazu wurde im Sumpf des Flashverdampfers über einen Wärmetauscher geringfügig nachgeheizt. Die aufkonzentrierte Absäure (Strom 3) wurde der Reaktion erneut zugeführt.

Die im Flashverdampfer C abgetrennten Brüden (35,7 kg/h, Strom 8) wurden anschließend in dem Wärmetauscher D kondensiert. Aufgrund des hohen MNT-Gehalts von 78 Gew.-%, bezogen auf den Organikaanteil im Brüdenstrom, bildeten sich in dem Brüdenkondensator D keine Ablagerungen.

Die im Phasentrenner B abgetrennten Organika (78,1 kg/h, Strom 5) wurden der zweiten Stufe (Reaktor E) zugeführt. Dort wurde in einem auf ca. 70°C gekühlten Schlaufenreaktor E durch Zugabe von Nitriersäure 96,0 kg/h DNT (Strom 13) hergestellt. Die dabei entstehende Absäure wurde in der Säureaufkonzentrierung F von überschüssigem Wasser befreit.

Durch die adiabate Reaktionsführung konnte in der ersten Stufe die Reaktionswärme zur Aufkonzentrierung der Absäure genutzt werden. Im Vergleich zu einer isotherm durchgeführten Mononitrierung ergab sich für den Gesamtprozess dadurch eine Heizdampf-Einsparung von ca. 40%. Sicherheitstechnische Untersuchungen des Reaktionsgemisches (Strom 4) bei der adiabaten Reäktionsendtemperatur von 131°C ergaben keinerlei Hinweise auf eine beginnende Zersetzung. Auch bei einem Belastungszeitraum von über 90 Minuten zeigten sich keine sicherheitstechnisch bedenklichen exothermen Reaktionen.

### Beispiel 2

Es wurde eine Apparatur gemäß Figur 2 verwendet. Die Temperaturen und Zusammensetzungen der Stoffströme sind in Tabelle 2 zusammengefasst.

In der adiabat betriebenen ersten Stufe wurden 50,6 kg/h Toluol (Strom 21), 62,9 kg/h 68 gew.-%ige Salpetersäure (Strom 22) und 736,0 kg/h 78,6 gew.-%ige aufkonzentrierte Absäure (Strom 23) am Eingang des Reaktors A (Abmessungen L = 8 m, D = 80 mm) intensiv gemischt. In dem mit Lochscheiben zur Redispergierung ausgerüstete Rohrreaktor stieg die Temperatur auf 138°C. Das Reaktionsgemisch (Strom 24) wurde über eine Düse in den bei 40 mbar betriebenen Flashverdampfer C entspannt, wo ein Teil des Wassers und der organischen Bestandteile verdampfte. Die Brüden wurden anschließend in dem Wärmetauscher D kondensiert. Trotz Kühlung mit 18°C kaltem Wasser bildeten sich in dem Brüdenkondensator D keine Ablagerungen. Das Brüdenkondensat (Strom 29) wurde in Phasentrenner B2 in die wässrige Phase (21,3 kg/h, Strom 30) und organische Phase (57,6 kg/h, Strom 31) aufgetrennt. Die überwiegend aus MNT bestehende organische Phase wurde der zweiten Stufe (Reaktor E) zugeführt.

Das durch Entspannungsverdampfung auf 94°C abgekühlte Säure/DNT/MNT-Gemisch (Strom 25) wurde in Phasentrenner B1 von ungelöstem DNT/MNT befreit. Das abgetrennte Gemisch (20,2 kg/h, Strom 26) wurde zusammen mit Strom 31 der Dinitrierung E zugeführt. Von der auf 77,1 Gew.-% aufkonzentrierten Absäure (750,4 kg/h, Strom 27) wurde ein Teilstrom (83,8 kg/h, Strom 32) der Säureaufkonzentrierung F in der zweiten Stufe zugeführt, dort auf 93 Gew.-% aufkonzentriert und dann in den Säurekreislauf der ersten Stufe zurückgegeben (Strom 33).

In der Dinitrierung E wurden in einem auf ca. 70°C gekühlten Schlaufenreaktor aus den beiden Zuläufen 31 und 26 durch Zugabe von Nitriersäure 96,1 kg/h DNT (Strom 13) hergestellt. Die dabei entstehende Absäure wurde in der Säureaufkonzentrierung F vom überschüssigen Wasser befreit.

Durch die adiabate Reaktionsführung in der ersten Stufe konnte ein erheblicher Teil des eingebrachten Wassers ohne Zuführung von Fremdenergie abgetrennt werden.

### Beispiel 3

Es wurde eine Apparatur gemäß Figur 3 verwendet. Die Temperaturen und Zusammensetzungen der Stoffströme sind in Tabelle 3 zusammengefasst. In der adiabat betriebenen ersten Stufe wurden 50,6 kg/h Toluol (Strom 21), 63,0 kg/h 68 gew.-%ige Salpetersäure (Strom 22) und 1306,6 kg/h 82,4 gew.-%ige aufkonzentrierte Absäure (Strom 23) am Eingang des Reaktors A (Abmessungen L = 5 m, D = 80 mm) intensiv gemischt. In dem mit Lochscheiben zur Redispergierung ausgerüstete Rohrreaktor stieg die Temperatur auf 132°C. Das Reaktionsgemisch (Strom 24) wurde über eine Düse in den bei 40 mbar betriebenen Flashverdampfer C entspannt, wo ein Teil des Wassers und der organischen Bestandteile verdampfte. Die Brüden wurden anschließend in dem Wärmetauscher D kondensiert. Trotz Kühlung mit 18°C kaltem Wasser bildeten sich in dem Brüdenkondensator D keine Ablagerungen. Das Brüdenkondensat (Strom 29) wurde in Phasentrenner B2 in die wässrige Phase (18,3 kg/h, Strom 30) und organische Phase (56,4 kg/h, Strom 41) aufgetrennt.

Das durch Entspannungsverdampfung auf 109°C abgekühlte Säure/DNT/MNT-Gemisch (Strom 25) wurde in Phasentrenner B1 von ungelöstem DNT/MNT befreit. Das abgetrennte Gemisch (21,4 kg/h, Strom 26) wurde der zweiten Stufe (Reaktor E) zugeführt. Von der auf 81,3 Gew.-% aufkonzentrierten Absäure (1324,1 kg/h, Strom 27) wurde ein Teilstrom (137,5 kg/h, Strom 32) der Säureaufkonzentrierung F in der zweiten Stufe zugeführt, dort auf 93 Gew.-% aufkonzentriert und dann in den Säurekreislauf der ersten Stufe zurückgegeben (Strom 33).

In der zweiten Stufe wurden in einem auf ca. 70°C gekühlten Schlaufenreaktor E aus den beiden Zuläufen 42 und 26 durch Zugabe von Nitriersäure 96,2 kg/h DNT (Strom 13) hergestellt. Aus der dabei entstehenden Absäure (Strom 43) wurde in der Extraktion G mit organischem Brüdenkondensat aus der ersten Stufe (Strom 41) ein Teil des gelösten DNT's entfernt. Anschließend wurde die Absäure (Strom 44) in der Säureaufkonzentrierung F von überschüssigem Wasser befreit. Die organische Phase aus der Extraktion G (59,4 kg/h, Strom 42) wurde zusammen mit Strom 26 der Dinitrierung E zugeführt.

Durch die adiabate Reaktionsführung in der ersten Stufe konnte ein erheblicher Teil des eingebrachten Wassers ohne Zuführung von Fremdenergie abgetrennt werden.

Die Temperaturen und Zusammensetzungen der in Beispiel 1-3 beschriebenen Ströme sind in den Tabellen 1-3 aufgelistet.

**Tabelle 1**

| **Stoffstrom-Nr.** | | **1** | **2** | **3** | **4** | **5** | **6** | **8** | **13** |
|---|---|---|---|---|---|---|---|---|---|
| Temperatur | °C | 20,0 | 20,0 | 100,0 | 131,3 | 131,3 | 131,3 | 100,0 | 68,0 |
| | | | | | | | | | |
| Gesamtstrom | kg/h | 50,59 | 62,98 | 1066,60 | 1180,17 | 78,10 | 1102,08 | 35,68 | 101,55 |
| Toluol | kg/h | 50,59 | | | | | | | |
| MNT | kg/h | | | 0,12 | 57,73 | 55,13 | 2,60 | 2,48 | |
| DNT | kg/h | | | 1,81 | 25,29 | 22,76 | 2,53 | 0,72 | 95,98 |
| H₂SO₄ | kg/h | | | 817,25 | 817,25 | 0,16 | 817,08 | | 1,11 |
| HNO₃ | kg/h | | 42,83 | 0,01 | 0,12 | | 0,12 | 0,11 | |
| H₂O | kg/h | | 20,15 | 247,42 | 279,79 | 0,04 | 279,74 | 32,37 | 0,44 |

**Tabelle 2**

| **Stoffstrom-Nr.** | | **13** | **21** | **22** | **23** | **24** | **25** | **26** |
|---|---|---|---|---|---|---|---|---|
| Temperatur | °C | 68,0 | 20,0 | 20,0 | 94,2 | 138,3 | 94,4 | 94,4 |
| | | | | | | | | |
| Gesamtstrom | kg/h | 101,01 | 50,59 | 62,94 | 735,96 | 849,48 | 770,64 | 20,21 |
| Toluol | kg/h | | 50,59 | | | | | |
| MNT | kg/h | | | | 0,13 | 57,74 | 3,19 | 3,05 |
| DNT | kg/h | 96,12 | | | 1,70 | 25,18 | 19,07 | 17,17 |
| H₂SO₄ | kg/h | 0,66 | | | 577,22 | 577,22 | 577,21 | |
| HNO₃ | kg/h | | | 42,80 | 0,01 | 0,08 | 0,01 | |
| H₂O | kg/h | 0,34 | | 20,14 | 156,90 | 189,26 | 171,16 | |
| | | | | | | | | |

| **Stoffstrom-Nr.** | | **27** | **29** | **30** | **31** | **32** | **33** | |
|---|---|---|---|---|---|---|---|---|
| Temperatur | °C | 94,4 | 27,3 | 27,3 | 27,3 | 94,4 | 40,0 | |
| | | | | | | | | |
| Gesamtstrom | kg/h | 750,43 | 78,84 | 21,25 | 57,59 | 83,80 | 68,51 | |
| Toluol | kg/h | | | | | | | |
| MNT | kg/h | 0,14 | 54,55 | 2,46 | 52,09 | 0,02 | | |
| DNT | kg/h | 1,91 | 6,11 | 0,61 | 5,50 | 0,21 | 0,00 | |
| H₂SO₄ | kg/h | 577,21 | | | | 64,46 | 63,67 | |
| HNO₃ | kg/h | 0,01 | 0,08 | 0,08 | | | | |
| H₂O | kg/h | 171,16 | 18,11 | 18,11 | | 19,11 | 4,83 | |

**Tabelle 3**

| | | | | | | | | | |
|---|---|---|---|---|---|---|---|---|---|
| | | | | | | | | | |

| **Stoffstrom-Nr.** | | **13** | **21** | **22** | **23** | **24** | **25** | **26** | **27** |
|---|---|---|---|---|---|---|---|---|---|
| Temperatur | °C | 68,0 | 20,0 | 20,0 | 104,3 | 132,3 | 109,4 | 109,4 | 109,4 |
| | | | | | | | | | |
| Gesamtstrom | kg/h | 101,01 | 50,59 | 63,01 | 1306,59 | 1420,19 | 1345,50 | 21,44 | 1324,07 |
| Toluol | kg/h | | 50,59 | | | | | | |
| MNT | kg/h | | | | 0,18 | 57,79 | 4,53 | 4.32 | 0,21 |
| DNT | kg/h | 96,19 | | | 1,71 | 25,21 | 19,02 | 17,11 | 1,90 |
| H₂SO₄ | kg/h | 0,66 | | | 1074,44 | 1074,44 | 1074,43 | | 1074,43 |
| HNO₃ | kg/h | | | 42,85 | 0,02 | 0,14 | 0,02 | | 0,02 |
| H₂O | kg/h | 0,34 | | 20,16 | 230,24 | 262,62 | 247,51 | | 247,51 |
| | | | | | | | | | |

| **Stoffstrom-Nr.** | | **29** | **30** | **32** | **33** | **41** | **42** | **43** | **44** |
|---|---|---|---|---|---|---|---|---|---|
| Temperatur | °C | 27,3 | 27,3 | 109,4 | 40,0 | 27,3 | 54,4 | 68,0 | 54,4 |
| | | | | | | | | | |
| Gesamtstrom | kg/h | 74,69 | 18,26 | 137,47 | 119,01 | 56,43 | 59,40 | 235,97 | 233,00 |
| Toluol | kg/h | | | | | | | | |
| MNT | kg/h | 53,26 | 2,40 | 0,02 | | 50,86 | 42,72 | | 8,14 |
| DNT | kg/h | 6,19 | 0,62 | 0,20 | 0,00 | 5,57 | 16,68 | 14,29 | 3,18 |
| H₂SO₄ | kg/h | 0,01 | 0,01 | 111,55 | 110,61 | | | 187,70 | 187,70 |
| HNO₃ | kg/h | 0,12 | 0,12 | | | | | 0,01 | 0,01 |
| H₂O | kg/h | 15,11 | 15,11 | 25,70 | 8,39 | | | 33,97 | 33,97 |

## Patentansprüche

1. Verfahren zur Herstellung von Dinitrotoluol durch zweistufige Nitrierung von Toluol, bei dem man
a) in einer ersten Stufe Toluol mit Nitriersäure adiabatisch umsetzt, wobei das Toluol zu mindestens 90 % abreagiert und wobei maximal 70 % des eingesetzten Toluols zu Dinitrotoluol reagieren, anschließend die Mononitrotoluol enthaltende organische Phase und die wässrige, Schwefelsäure enthaltende Säurephase trennt, die wässrige, Schwefelsäure enthaltende Säurephase durch Entspannungsverdampfung aufkonzentriert und die dabei erhaltene aufkonzentrierte Schwefelsäure in die - Reaktion der ersten Stufe und/oder die Reaktion der zweiten Stufe und/oder die Aufkonzentrierung der zweiten Stufe zurückführt, und
b) in einer zweiten Stufe die Mononitrotoluol enthaltende organische Phase aus der ersten Stufe mit Nitriersäure isotherm vollständig umsetzt, anschließend die organische Phase und die wässrige, Schwefelsäure enthaltende Säurephase trennt und die wässrige, Schwefelsäure enthaltende Säurephase durch Vakuumverdampfung aufkonzentriert und die dabei erhaltene aufkonzentrierte Schwefelsäure in die Reaktion der ersten Stufe und/oder der zweiten Stufe zurückführt.

2. Verfahren nach Anspruch 1, bei dem man die organische, Mononitrotoluol enthaltende Phase und die wässrige, Schwefelsäure enthaltende Säurephase aus der ersten Stufe zuerst gemeinsam durch Entspannungsverdampfung aufkonzentriert und dann die organische, Mononitrotoluol enthaltende Phase und die wässrige, Schwefelsäure enthaltende Säurephase trennt.

3. Verfahren nach einem der Ansprüche 1 oder 2, bei dem man die wässrige, Schwefelsäure enthaltende Säurephase aus der zweiten Stufe vor der Vakuumverdampfung mit einer organischen, Mononitrotoluol enthaltenden Phase aus der ersten Stufe extrahiert.

## Claims

1. Process for preparing dinitrotoluene by two-stage nitration of toluene, wherein
a) in a first stage, toluene is reacted adiabatically with nitrating acid, with the toluene reacting to an extent of at least 90% and not more than 70% of the toluene used reacting to form dinitrotoluene, the organic phase containing mononitrotoluene and the aqueous acid phase containing sulphuric acid are subsequently separated, the aqueous acid phase containing sulphuric acid is concentrated by flash evaporation and the more concentrated sulphuric acid obtained is recirculated to the reaction of the first stage and/or the reaction of the second stage and/or the concentration operation of the second stage, and
b) in a second stage, the organic phase containing mononitrotoluene from the first stage is completely reacted isothermally with nitrating acid, the organic phase and the aqueous acid phase containing sulphuric acid are subsequently separated and the aqueous acid phase containing sulphuric acid is concentrated by vacuum evaporation and the more concentrated sulphuric acid obtained is recirculated to the reaction of the first stage and/or of the second stage.

2. Process according to Claim 1, wherein the organic phase containing mononitrotoluene and the aqueous acid phase containing sulphuric acid from the first stage are firstly concentrated together by flash evaporation and the organic phase containing mononitrotoluene and the aqueous acid phase containing sulphuric acid are then separated.

3. Process according to either Claim 1 or 2, wherein the aqueous acid phase containing sulphuric acid from the second stage is extracted with an organic phase containing mononitrotoluene from the first stage before the vacuum evaporation.

## Revendications

1. Procédé pour la production de dinitrotoluène par nitration du toluène en deux étapes, dans lequel
a) dans une première étape on fait réagir en mode adiabatique du toluène avec du mélange sulfonitrique, le toluène réagissant à raison d'au moins 90 % et au maximum 70 % du toluène introduit étant convertis en dinitrotoluène, ensuite on sépare la phase organique contenant du mononitrotoluène et la phase aqueuse acide contenant de l'acide sulfurique, on concentre par évaporation par détente la phase aqueuse acide contenant de l'acide sulfurique et l'acide sulfurique concentré ainsi obtenu est recyclé dans la réaction de la première étape et/ou la réaction de la deuxième étape et/ou la concentration de la deuxième étape, et
b) dans la deuxième étape on fait réagir complètement en mode isothermique avec du mélange sulfonitrique le phase organique contenant du mononitrotoluène, provenant de la première étape, ensuite on sépare la phase organique et la phase aqueuse acide contenant de l'acide sulfurique et on concentre par évaporation sous vide la phase aqueuse acide contenant de l'acide sulfurique et on recycle l'acide sulfurique concentré, ainsi obtenu, dans la réaction de la première étape et/ou de la deuxième étape.

2. Procédé selon la revendication 1, dans lequel d'abord on concentre ensemble par évaporation par détente la phase organique contenant du mononitrotoluène et la phase aqueuse acide contenant de l'acide sulfurique, provenant de la première étape, et ensuite on sépare la phase organique contenant du mononitrotoluène et la phase aqueuse acide contenant de l'acide sulfurique.

3. Procédé selon l'une quelconque des revendications 1 et 2, dans lequel avant l'évaporation sous vide on extrait avec une phase organique contenant du mononitrotoluène, provenant de la première étape, la phase aqueuse acide contenant de l'acide sulfurique, provenant de la deuxième étape.
